Europäisches Patentamt

European Patent Office

Office européen des brevets

(11) Veröffentlichungsnummer: **0 358 809**
**A1**

(12) # EUROPÄISCHE PATENTANMELDUNG

(21) Anmeldenummer: 88115151.8

(51) Int. Cl.5: **G01N 21/31 , A61B 5/00**

(22) Anmeldetag: **15.09.88**

(43) Veröffentlichungstag der Anmeldung:
**21.03.90 Patentblatt 90/12**

(84) Benannte Vertragsstaaten:
**CH DE FR GB LI**

(71) Anmelder: **Hellige GmbH**
**Postfach 728 Heinrich-von-Stephan-Strasse 4**
**D-7800 Freiburg im Breisgau(DE)**

(72) Erfinder: **Waidelich, Wilhelm, Prof. Dr. Institut für Med.**
**Optik der Universität München**
**Barbarastrasse 16**
**D-8000 München 40(DE)**
Erfinder: **Spahn, Jürgen, Dipl.-Phys. Institut für Med.**
**Optik der Universität München**
**Barbarastrasse 16**
**D-8000 München 40(DE)**
Erfinder: **Essenpreis, Matthias Institut für Med.**
**Optik der Universität München**
**Barbarastrasse 16**
**D-8000 München 40(DE)**

(74) Vertreter: **Patentanwälte TER MEER - MÜLLER - STEINMEISTER**
**Mauerkircherstrasse 45**
**D-8000 München 80(DE)**

(54) **Spektralphotometer zur Messung am lebenden Organismus.**

(57) Das Spektralphotometer zur Messung am lebenden Organismus enthält erfindungsgemäß als eine im Pulsbetrieb steuerbare Lichtquelle (1) eine mit Krypton gefüllte Gasentladungslampe, welche den Vorteil hat, daß ihr Emissionsspektrum weitgehend übereinstimmt mit den Lichtwellenlängen, die für die Messung insbesondere der Vitalgrößen Cytochrom $aa_3$ und Oxygenierung von Hämoglobin benötigt werden.

EP 0 358 809 A1

Fig. 9

EP 0 358 809 A1

## Spektralphotometer zur Messung am lebenden Organismus

Die Erfindung betrifft ein Spektralphotometer nach dem Oberbegriff des Patentanspruchs 1, das zur nichtinvasiven diagnostischen Untersuchung oder Überwachung am lebenden Organismus, insbesondere auch am Menschen, geeignet ist.

In der medizinischen Diagnostik nehmen seit einigen Jahren nichtinvasive Methoden einen immer größer werdenden Stellenwert ein. Große Fortschritte sind dabei auch bei der Transillumination und der Absorptionsspektroskopie von Gewebe und Organen erzielt worden. Durch solche Untersuchungen lassen sich aus der gemessenen Absorption Werte für die Oxygenierung von Hämoglobin (Sauerstoffsättigung) und den Redoxzustand von Cytochrom $aa_3$ ermitteln. Diese Messungen beruhen auf folgenden Voraussetzungen:

1. Menschliches Gewebe ist, wie bekannt, für Licht im nahen Infrarotbereich (ca. 700nm bis 950nm) relativ gut durchlässig. Dagegen machen im sichtbaren Bereich starke Absorptionsbanden des Hämoglobins und im fernen Infrarotbereich (ab ca. 1300nm) die Absorption durch Wasser ein tieferes Eindringen von elektromagnetischer Strahlung unmöglich.

2. Sehr wenige biologische Substanzen absorbieren im nahen Infrarotbereich, so daß Überlagerungen der Absorption nicht zu erwarten sind.

3. Hämoglobin und Cytochrom $aa_3$ sind nach derzeitigem Wissensstand die einzigen, im nahen Infrarot absorbierenden Substanzen, die meßbar auf unterschiedliche Oxygenierung bzw. Oxidation reagieren. Daher sind sie als direkte Indikatoren des Sauerstoffgehalts im Gewebe geeignet.

Absorptionsmessungen am Gewebe in diesem Bereich liefern demnach Aussagen über zwei extrem wichtige Vitalgrößen der Intensivmedizin: die Oxygenation des Bluts und den Redoxzustand des Cytochrom $aa_3$ im gemessenen Gewebevolumen.

Zum Stand der Technik wird auf folgende Literaturstellen hingewiesen: (1) JÖBSIS, F.F.: Noninvasive, Infrared Monitoring of Cerebral and Myocardial Oxygen Sufficiency and Circulatory Parameters. Science 198, 1264 bis 1267 (1977); (2) DE-C2- 30 19 234 und (3) DE-A1-35 28 369.

Die Erfindung geht von dem in der Literaturstelle (3) beschriebenen Stand der Technik aus, aus dem es bekannt ist, gepulstes Licht mehrerer Wellenlängen zum Messen von Blutkreislauf- und örtlichen Stoffwechselparametern im lebenden Organismus zu benutzen. Beschrieben ist dort insbesondere die Strahlungsausbreitung von mindestens vier signifikanten Wellenlängen zu erfassen und als Lichtquelle eine Xenon-Blitzlampe zu verwenden.

Will man mit den in (1), (2) und (3) beschriebenen Methoden zur quantitativen und damit für die Medizin verläßlichen Aussagen über den Redoxzustand des Cytochrom $aa_3$ gelangen, ohne jedoch das untersuchte Gewebe zu schädigen, so gibt es auch mit dem in (3) beschriebenen Gerät extreme Schwierigkeiten, insbesondere wenn man eine längerdauernde Überwachung beabsichtigt, wie sie in der Intensivmedizin gefordert wird. Der Grund liegt in der erforderlichen hohen Strahldichte des eingestrahlten Lichts. Diese ist nötig, weil die starke Streuung des Lichts im Gewebe die Messung der Absorption in einen Bereich verschiebt, der außerhalb des linearen Zusammenhangs des Lambert-Beer'schen Gesetzes liegt.

Der Erfindung liegt damit die Aufgabe zugrunde, ein Verfahren und eine Vorrichtung zu schaffen, durch die Messungen bei vergleichsweise möglichst kleinen Leuchtdichten möglich sind, um die Gewebebelastung so gering wie möglich zu halten. Gleichzeitig sollten für die medizinische Diagnose aussagekräftige und quantitativ hinreichend genaue Meßwerte geliefert werden.

Die Erfindung ist bei einem Spektralphotometer zur Messung am lebenden Organismus durch die Merkmale des Patentanspruchs 1 gegeben.

Vorteilhafte Weiterbildungen des Erfindungsgedankens sind in abhängigen Patentansprüchen sowie auch in der nachfolgenden Beschreibung mit Bezug auf Zeichnungen näher erläutert.

Die Untersuchungen zur Lösung der angegebenen Aufgabe führten zu der überraschenden Feststellung, daß das Emissionsspektrum von Krypton in erstaunlich guter Übereinstimmung gerade das Licht der Wellenlängen liefert, die für diese Messungen gebraucht werden. Erfindungsgemäß wird daher eine mit Krypton gefüllte Gasentladungslampe, vorzugsweise eine Kryptonblitzlampe verwendet, wie sie in keiner der bisher beschriebenen Realisierungen angewendet oder in Betracht gezogen wurde. Es war offensichtlich unbekannt, daß die Emissionsmaxima einer Kryptonlampe außerordentlich gut mit den zur Realisierung der Meßmethode benötigten Wellenlängen übereinstimmen.

Eine mit Krypton gefüllte Gasentladungslampe erfüllt überraschend gut die Anforderungen, die an eine als für das Verfahren geeignet zu bezeichnende Lichtquelle gestellt werden. Die gute Übereinstimmung der relativ schmalen Emissionsbanden der Kryptonlampe mit den Absorptionsmaxima der zu untersuchenden Substanzen läßt den Einsatz kostengünstiger und transmissionsstarker, breitbandiger Interferenzfilter zu

2

EP 0 358 809 A1

oder die Verwendung zweier geeigneter Kantenfilter, die das benötigte optische Fenster begrenzen.

Mit der Erfindung ergeben sich einige weitere Vorteile:

- Die thermische Belastung der Filterelemente wird niedrig gehalten.

- Eine thermische Wellenlängenverschiebung, wie sie bei schmalbandigen Interferenzfiltern in Verbindung mit Lichtquellen mit kontinuierlichem Spektrum auftritt und zu Drifterscheinungen führt, wird ausgeschlossen, da die Meßwellenlängen im wesentlichen durch das Emissionsspektrum der Kryptonlampe festgelegt sind.

Im folgenden wird die Erfindung mit weiteren vorteilhaften Einzelheiten anhand von Schaubildern und Blockschaltbildern sowie Prinzipzeichnungen näher erläutert. Es zeigen:

Fig. 1 ein Schaubild von Absorptionsspektren von Hämoglobin mit variabler Oxygenierung als Parameter; die Absorption fällt mit zunehmender Oxygenierung; der isosbestische Punkt liegt bei ca. 810nm;

Fig. 2 ein Schaubild des Absorptionsspektrums von Cyto chrom $aa_3$; oxidiert (A), reduziert (B);

Fig. 3 das Emissionsspektrum einer Kryptonblitzlampe (Hersteller: EG & G);

Fig. 4 das Emissionsspektrum einer Kryptonstablampe (Hersteller: OSRAM);

Fig. 5(A) und 5(B) das Blockdiagramm einer Meßapparatur einmal mit Photomultiplier (Fig. 5(A)) und einmal mit Avalanche-Dioden (Fig. 5(B)) als Detektorsystem;

Fig. 6(A) die schematische Perspektivdarstellung eines optischen Aufbaus;

Fig. 6(B) die Schnittdarstellung durch die Ebene der optischen Achse eines optischen Aufbaus mit Lichtquelle;

Fig. 7 eine optische Sensoranordnung zur Einkopplung des Lichts in das zu untersuchende Gewebe;

Fig. 8(A) eine optische Sensoranordnung mit Lichtleiter als Aufnehmer;

Fig. 8(B) eine optische Sensoranordnung mit Avalanche-Dioden als Detektoren;

Fig. 9 einen vollständigen Meßaufbau mit einem gegenüber dem Prinzipblockschaltbild der Fig. 5(A) genauer spezifizierten Blockschaltbild einschließlich des "Meßobjekts" und der Auswertegeräte;

Fig. 10 die graphische Darstellung von Meßergebnissen bei der Bestimmung von Absorptionsänderungen im menschlichen Gewebe;

Fig. 11 die graphische Darstellung des Meßergebnisses von Änderungen der Konzentrationen von oxygeniertem Hämoglobin (HbO) und deoxygeniertem Hämoglobin (HbR); und

Fig. 12 die photographische Wiedergabe eines Oszillographenbildschirms zur Veranschaulichung der zeitlichen Zusammenhänge bei einem Meßablauf unter Einblendung eines Zeitfensters.

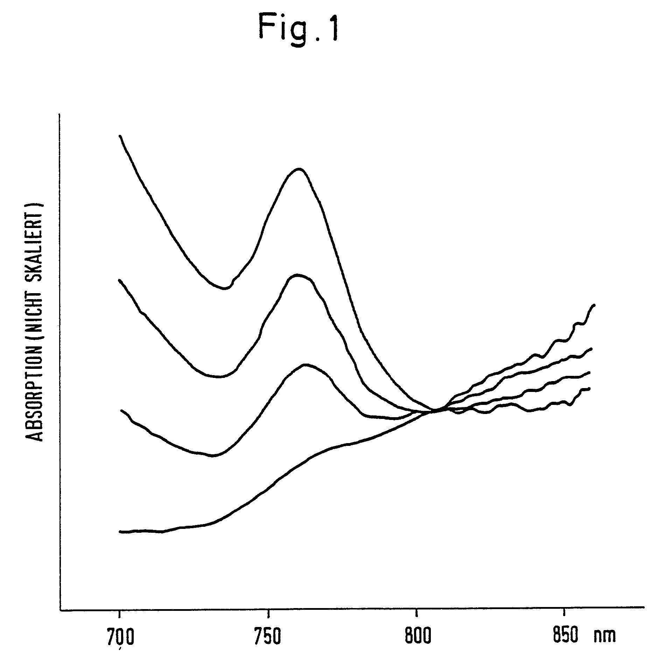

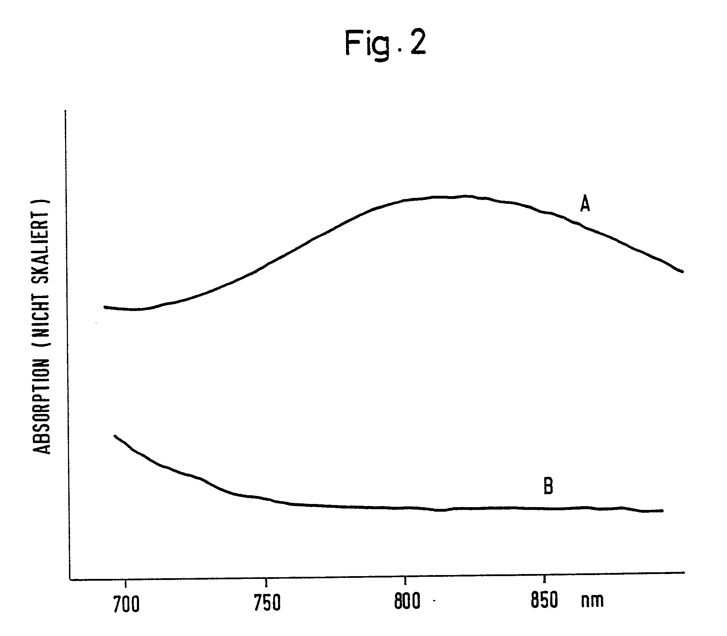

Wie in (2) beschrieben, werden bei der zugrunde liegenden Meßmethode mindestens drei Meßpunkte ausgewertet. Diese wählt man geeigneterweise in einem Bereich großer Absorptionsunterschiede der zu untersuchenden Substanzen insbesondere bei 760nm für oxygeniertes bzw. desoxygeniertes Hämoglobin (siehe Fig. 1) und bei 850nm für Cytochrom $aa_3$ (siehe Fig. 2). Als dritten Meßpunkt benötigt das Verfahren noch mindestens einen Bezugswert, um unerwünschte Veränderungen der Absorption zu eliminieren. Hier kommt insbesondere der Wert von 810nm als isosbestischer Punkt für Hämoglobin/Oxyhämoglobin in Frage für die Messung der im Absorptionsvolumen vorhandenen Blutmenge, die im Verlauf der Messungen Veränderungen unterliegen kann.

Beim Verfahren gemäß der Erfindung werden vorzugsweise zusätzlich zwei weitere Bezugspunkte, insbesondere 870nm und 895nm, zur Berücksichtigung der Streuung und von Änderungen in der Einkopplung des Lichts ausgewertet.

Die zur Gewinnung der Meßwerte erforderlichen Detektoren sitzen erfindungsgemäß direkt am probenseitigen Ende der Lichtwellenleiter bzw. neben dem Ort der Einstrahlung des Lichts in das Gewebe.

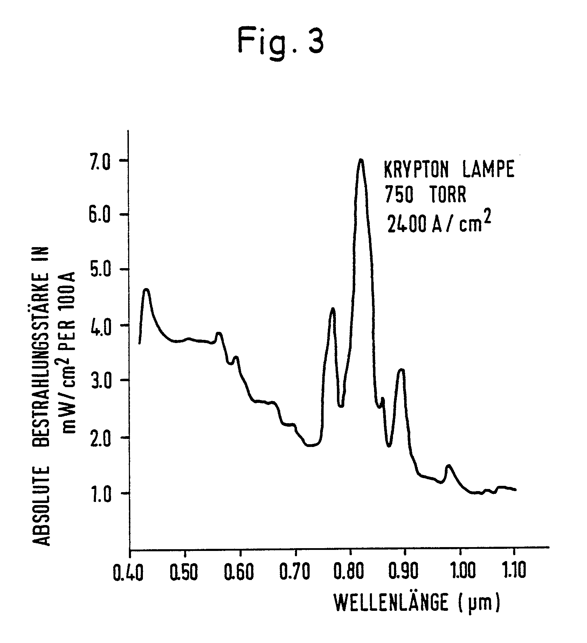

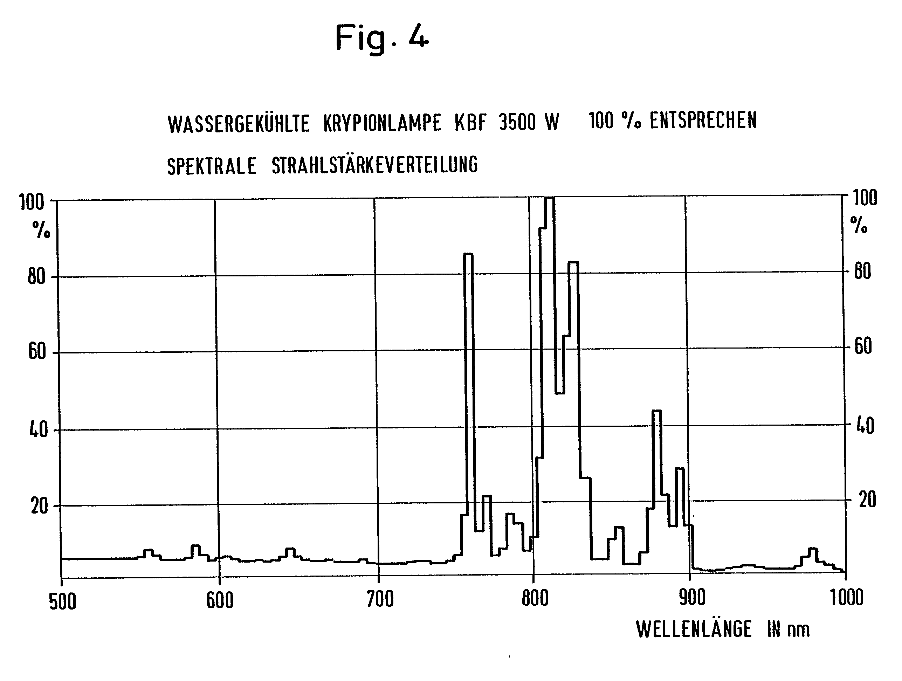

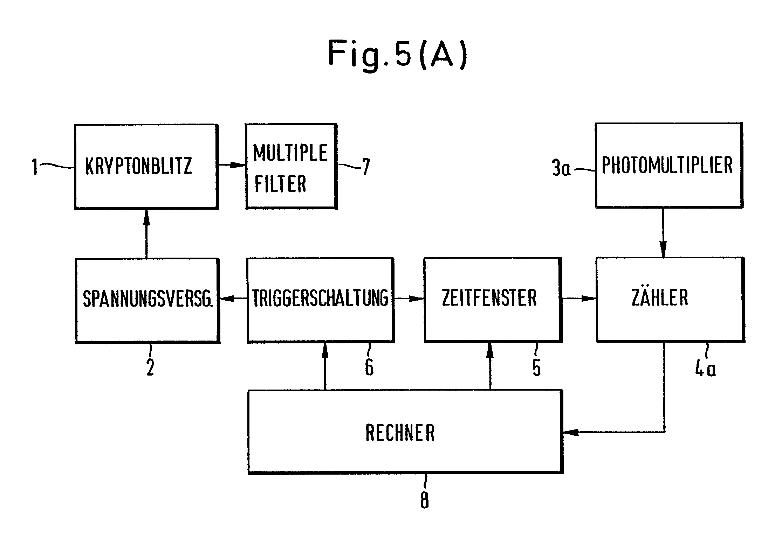

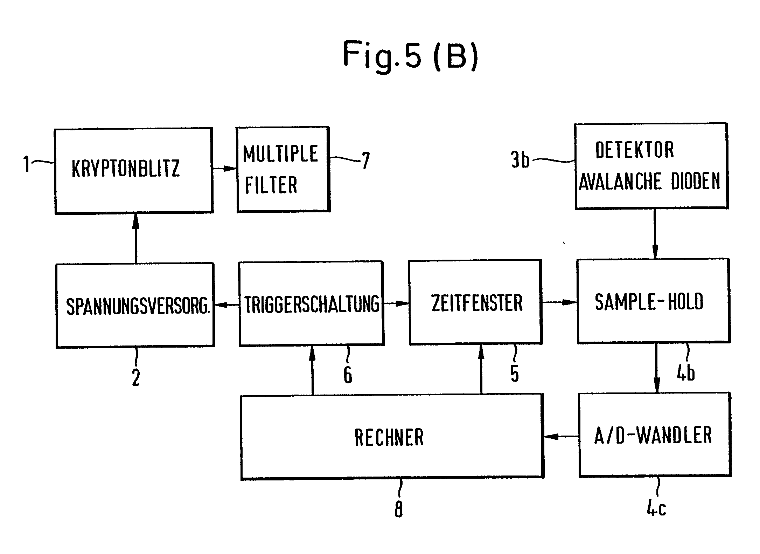

Blockschaltbilder und ergänzende Prinzipzeichnungen von Ausführungsbeispielen eines erfindungsgemäßen Spektralphotometers sind in den Fig. 5 bis 8 dargestellt. Als Lichtquelle wurde eine kommerziell erhältliche Kryptonblitzlampe der Firma EG & G, Typ XFK-272 verwendet, deren Emissionsspektrum in der Fig. 3 veranschaulicht ist. In Frage kommt jedoch auch die Verwendung einer stabförmigen Gasentladungslampe, z. B. des Typs KBF 3500 der Firma OSRAM, deren Lichtstrahl mittels eines Choppers geeignet moduliert wird und deren Emissionsspektrum durch die Fig. 4 veranschaulicht ist.

In den Blockschaltbildern der Fig. 5(A) und 5(B) haben die angegebenen Bezugszeichen die folgende Bedeutung:

1: Kryptonblitzlampe (z. B. EG & G, Typ XFK-272) als Lichtquelle. Diese Blitzlampe erlaubt Blitzenergien (elektrische Entladungsenergie pro Blitz) bis zu 100J. Damit ist eine mittlere Leistung pro Blitz von über 100kW mit einer optischen Leistung von ca. 20kW möglich.

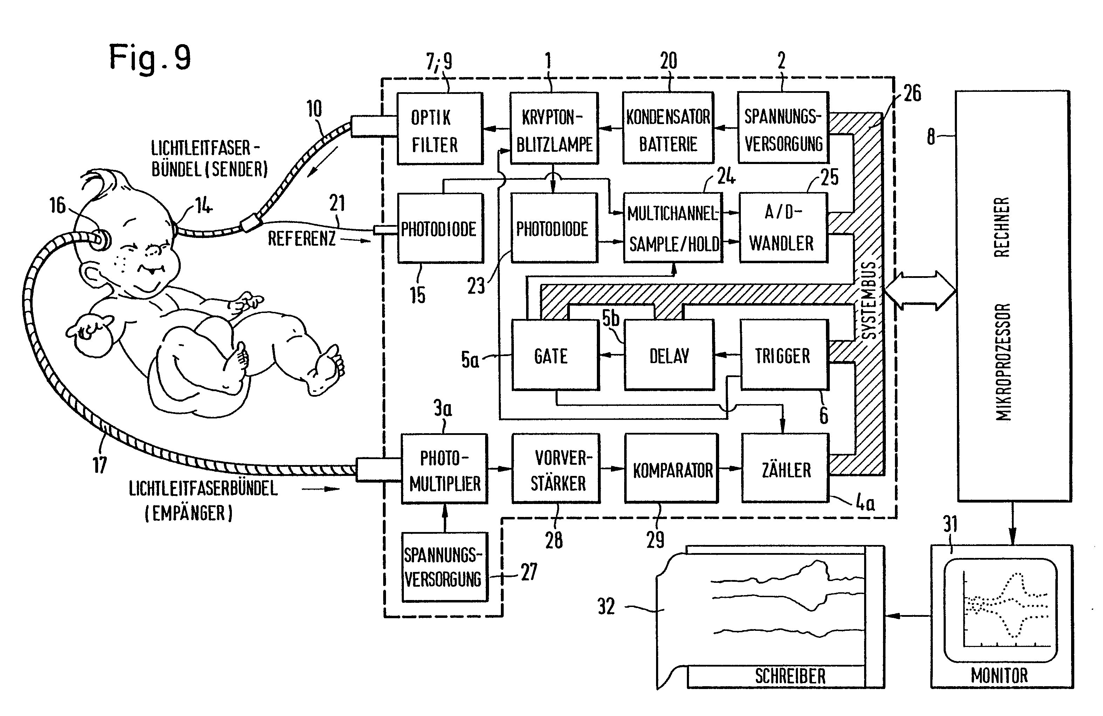

2: Spannungsversorgung für die Blitzlampe. Die Spannung wird zusammen mit den benötigten (Bezugshinweis 20 in Fig. 9) Blitzkondensatoren so vorgewählt, daß Blitzenergien von 0,01J bis 100J in Abständen von je einer Größenordnung einstellbar sind.

3

3a: In Fig. 5(A) wird als Lichtaufnahmeeinrichtung ein Photomultiplier, insbesondere des Typs RCA C31034 verwendet. Dieser Photomultiplier ist speziell für den Meßbereich im nahen Infrarot ausgelegt und hat dort sein Empfindlichkeitsmaximum. Um das störende thermische Rauschen zu senken, wird vorzugsweise außerdem eine (nicht dargestellte) Peltierkühlung eingesetzt.

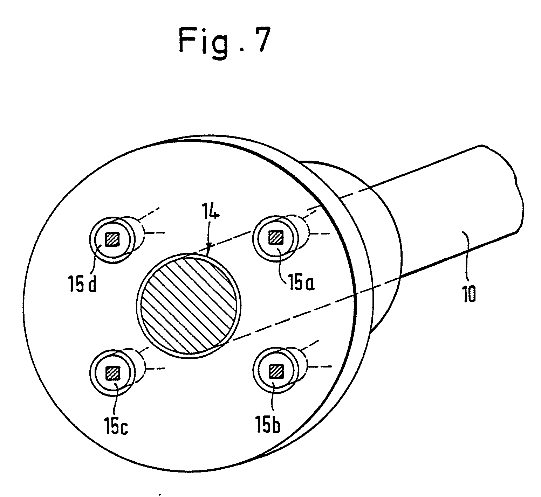

3b: Gemäß Fig. 5(B) können anstelle des Photomultipliers 3a in Fig. 5(A) mindestens eine, vorzugsweise jedoch mehrere, Avalanche-Dioden als Detektorsystem verwendet werden. Avalanche-Dioden haben eine ähnlich gute Photonenausbeute wie Photomultiplier (ca. 80 %). Ihr Einsatz bringt jedoch bedeutende Vorteile in der Handhabung. Insbesondere erlauben die geringen räumlichen Abmessungen von Avalanche-Dioden das Anbringen direkt auf und am Ort des zu untersuchenden Gewebes. Die Verwendung eines Lichtleiters und die damit verbundenen Verluste können somit vermieden werden. Ein Ausführungsbeispiel des optischen Sensoraufbaus unter Verwendung von Avalanche-Dioden wird weiter unten unter Bezug auf Fig. 7(B) erläutert.

Avalanche-Dioden haben darüber hinaus den Vorteil, daß sie keine Hochspannungsversorgung benötigen und bei Raumtemperatur betrieben werden können.

4a: In Fig. 5(A) ein Zähler. Um die hohe Empfindlichkeit des Photomultipliers ausnützen zu können, wird eine Photonenzählung durchgeführt. Die Weiterverarbeitung und Auswertung der gemessenen Impulszahlen erfolgt nach dem Transfer der Daten im Rechner 8.

4b: In Fig. 5(B) wird beim Einsatz von Avalanche-Dioden als Detektorsystem 3b der Zähler ersetzt durch eine Mehrkanal-Sample-Hold-Schaltung 4b. Diese wird in Zeitmultiplex über einen Flash-Analog-Digital-Wandler 4c ausgelesen. Nach dem Datentransfer erfolgt die Weiterverarbeitung wie in Fig. 5(A) im Rechner 8.

5: Elektronische Schaltung zur Festlegung eines Zeitfensters.
Um das Rauschen im Meßsignal weiter zu senken, werden der Photonenzähler 4a (Fig. 5(A)) bzw. die Sample- Hold-Schaltung 4b (Fig. 5(B)) nur während der tatsächlichen Blitzdauer aktiviert. Die Zeitdauer und die Zeitverzögerung nach der Triggerung der Blitzlampe werden vom Rechner 8 aus programmiert. Zeitdauer und Zeitverzögerung wurden in Versuchen experimentell für die verschiedenen Blitzenergien bestimmt.

Prinzipiell ist es möglich, das "Zeitfenster" zu beliebigen Zeitpunkten nach einer Triggerung zu starten. So erfolgt die eigentliche Messung beispielsweise mit einer Verzögerung von 6,5 $\mu$s und einer Auftast- oder Gate-Zeit von 20 $\mu$s. Bei Messungen wurden diese Werte experimentell als günstige Werte für eine Blitzenergie von 1J bestimmt.

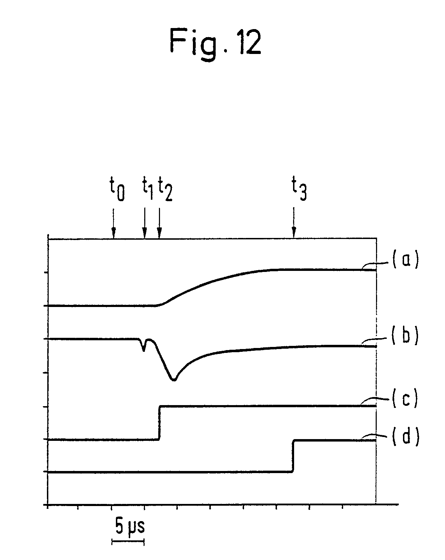

Die Fig. 12 zeigt die photographische Wiedergabe eines Oszillographenbildschirms, der den Meßablauf und die zeitliche Korrelation relevanter Signale in den Schaltungen nach den Fig. 5(A), 5(B) bzw. 9, insbesondere zur Festlegung des Zeitfensters (das "Timing") wiedergibt.

In dieser Fig. 12 bezeichnen die angegebenen Hinweise folgende Zeitpunkte bzw. Signalverläufe:
$t_0$: Triggerzeitpunkt ($t_0$ = 0 $\mu$s)
$t_1$: Zündzeitpunkt der Kryptonblitzlampe 1 ($t_1$ ~ 4,5 $\mu$s)
$t_2$: Start der Messung ($t_2$ = 5,6 $\mu$s)
$t_3$: Ende der Messung ($t_3$ = 26,5 $\mu$s)
(a): Ausgangsspannung am Integrator, beispielsweise des Photomultipliers 3a; normiert, Einheit: 1 V.
(b): Ausgangssignal der Photodiode, d. h. Ausgangsspannung des Vorverstärkers 28 in Fig. 9, Einheit: 1 V.
(c): Start der Messung (flankengetriggert), Einheit: 5 V.
(d): Ende (Stopp) der Messung (flankengetriggert), Einheit: 5 V.

An der Abszisse der Fig. 12 ist eine Vergleichszeiteinheit von 5 $\mu$s angegeben. Das Zeitfenster zwischen den Kurven (c) und (d) läßt ab dem Zeitpunkt $t_0$ eine Verzögerung (Delay) von 6,5 $\mu$s und eine Auftastzeit (Gatezeit) von ca. 20 $\mu$s erkennen. Die Energie des Blitzes der Kryptonblitzlampe 1 betrug ca. 1J.

Während der Zeit zwischen zwei Blitzen wird die Dunkelzählrate bei gleichem Zeitfenster wie für den vorhergehenden Blitz gemessen. Diese Dunkelzählrate ist zurückzuführen auf thermisches Rauschen des Photomultipliers 3a und insbesondere auf Streueffekte des Umgebungslichts im Gewebe. Die ermittelte Dunkelzählrate wi-rd von der zuvor während der Blitzdauer gemessenen Zählrate abgezogen.

6: Eine Triggerschaltung, gesteuert durch einen Impuls vom Rechner 8 legt den Zeitpunkt der Auslösung des Blitzes und den Zeitpunkt des Beginns des Zeitfensters unter Steuerung vom Rechner 8 aus gleichzeitig fest.

7: Ein Mehrfachfilter (Filterrad) enthält beispielsweise fünf verschiedene Interferenzfilter, wie sie etwa von der Firma SCHOTT bezogen werden können. Diese Interferenzfilter sind für folgende Wellenlängen ausgelegt: 7a für 760 nm; 7b für 810 nm; 7c für 850 nm; 7d für 870 nm, und 7e für 895 nm. Die Lage der Transmissionsmaxima dieser Interferenzfilter ist auf die Eigenschaften der Kryptonblitzlampe abgestimmt. Die Halbwertsbreite der Filter liegt zwischen 10 nm und 16 nm. Die automatische Umschaltung, also der Filterwechsel, erfolgt, gesteuert vom Rechner 8 aus, nach ca. 50 Blitzen.

8: Als Rechner 8 wird ein Mikroprozessorsystem verwendet. Erprobt wurde der Rechner unter der Typenbezeichnung AT von IBM, dessen Systembus an ein externes Gehäuse herausgeführt wurde. An diesem Systembus sind alle Meß- und Steuereinheiten, wie A/D-Wandler, Zähler, Zeitfenster, Trigger usw., angeschlossen.

Mit dem anhand der Blockschaltbilder der Fig. 5(A) und 9 bzw. 5(B) erläuterten Aufbau wurden zwei exemplarische Ausführungsbeispiele von Spektralphotometern erfindungsgemäßer Bauart erläutert. Das Gerät ist leicht bedienbar. Die zugehörige Software übernimmt neben der Kontrolle über die angeschlossenen Systeme auch die Aufbereitung und Auswertung der Daten. Diese werden dann an einem Monitor 31 und/oder (gleichzeitig) auf einem Schreiber 32 ausgegeben.

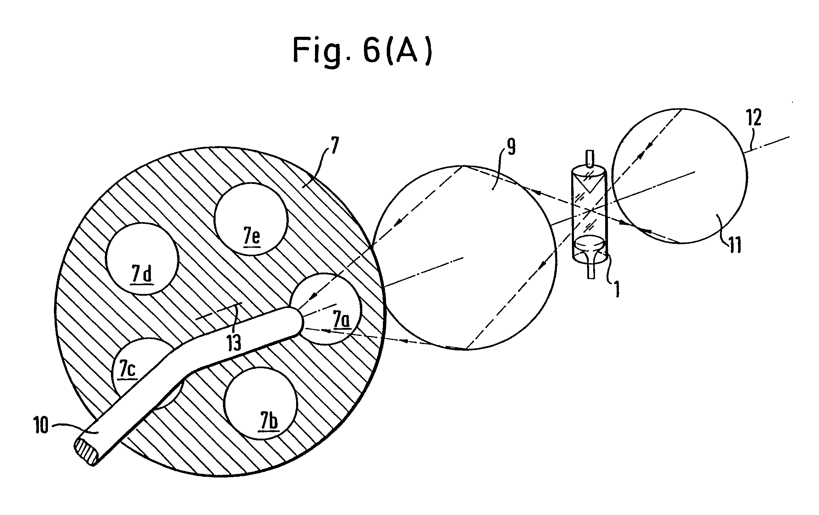

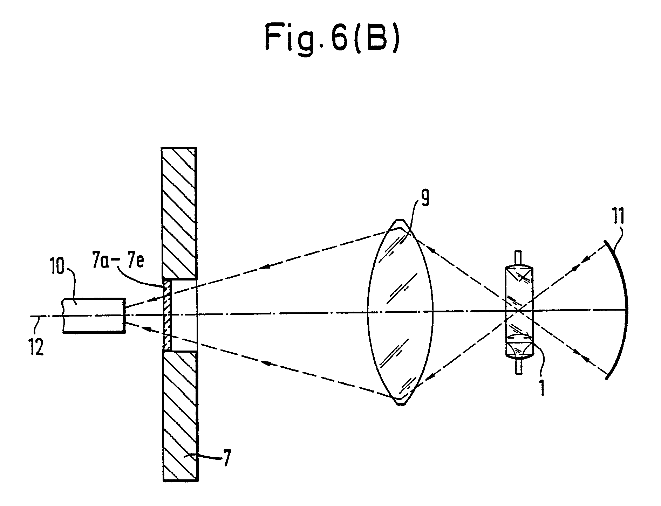

Der vollständige Aufbau des Meßsystems nach Fig. 9 läßt erkennen, daß der gesamte Meßablauf einschließlich der zeitrichtigen Steuerung der Darstellung der Meßkurven auf dem Monitor 31 bzw. dem Schreiber 32 vom Rechner 8 (Mikroprozessor) aus und mit Bezug auf das Meßobjekt (Probanden) über einen Systembus 26 erfolgt. Zunächst wird vom Rechner 8 entsprechend einer vom Bediener gewählten Verzögerungszeit über die Triggerschaltung 6 die Kryptonblitzlampe 1 ausgelöst, die ihre Energie von der Spannungsversorgung 2 über eine Kondensatorbatterie 20 erhält. Der Lichtblitz gelangt über die Optik 11, 9 und ein momentan ausgewähltes Filter 7 (vgl. Fig. 6(A)) auf das Lichtleitfaserbündel 10 zur Einkoppelstelle 14 (Senderseite). An der Einkoppelstelle 14 wird über eine oder mehrere Photozellen (Photodioden) 15 (vgl. Fig. 7) ein Referenzsignal gewonnen, das zusammen mit einem weiteren über eine von der Kryptonblitzlampe direkt bestrahlte Photodiode 23 gewonnenen Referenzsignal auf eine Mehrkanal-Sample-Hold-Schaltung 24 gelangt, deren Referenzausgangssignal über einen Analog/Digital-Wandler 25 und den Systembus 26 in den Rechner 8 gelangt. Die Sample-Hold-Schaltung 24 wird entsprechend dem über eine Verzögerungsstufe 5b (Delay) bestimmten Startzeitpunkt der Messung ein- bzw. nach Ablauf des durch eine Torschaltung 5a (Gate) bestimmten Zeitfensters wieder ausgeschaltet (gesperrt; Ende der Messung).

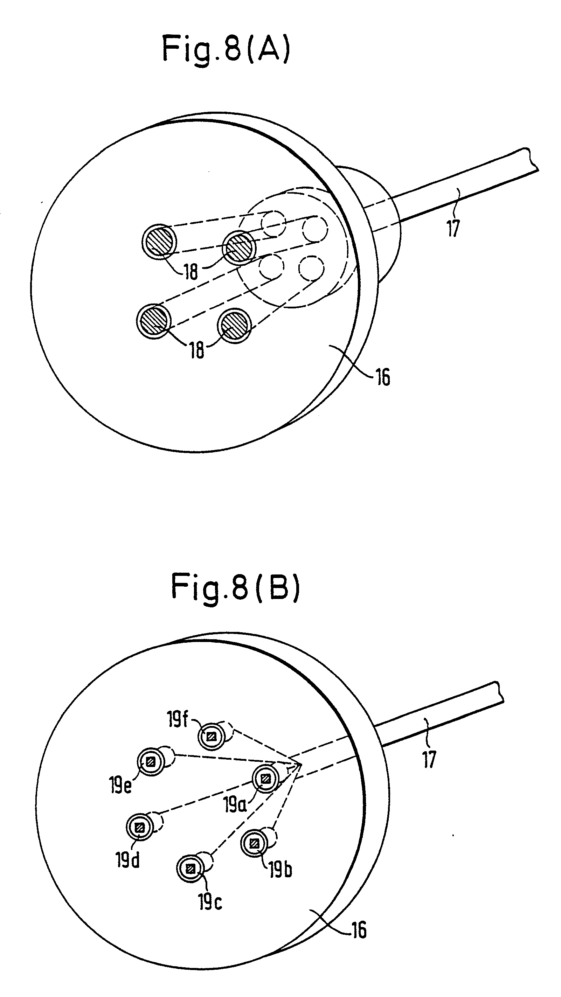

Auf der der Einkoppelstelle 14 gegenüberliegenden Seite des zu untersuchenden Gewebes wird ein optischer Aufnehmer (Empfänger) 16 angebracht, dessen Einzelheiten weiter unten unter Bezug auf die Fig. 8(A) erläutert werden. Die Signale des optischen Aufnehmers 16 gelangen über ein Lichtleitfaserbündel 17 auf den von einer Spannungsversorgung 27 gespeisten Photomultiplier 3a. Der Photomultiplier 3a ist über einen Vorverstärker 28 auf einen Komparator 29 geschaltet, der den Zähleingang eines Zählers 4a speist, welcher von der Torschaltung 5a am Ende der Messung zurückgesetzt wird.

Die nachfolgende Tabelle gibt das Strukturprogramm (Struktogramm) für das im Rechner 8 implementierte Meß- und Steuerungsprogramm wieder. Bei der praktischen Verwirklichung war ein Mikroprozessor IBM-AT verwendet worden und das Programm in TURBO-PASCAL geschrieben.

5

Struktogramm

| Namen der Datei zum Abspeichern der Meßwerte erfragen und eröffnen |
| --- |
| Messung vorbereiten (Einstellung der Meßparameter zu Beginn der Messung). |

> | DELAY einstellen (6.5µs) → Zeitverzögerung für Meßbeginn |
> | --- |
> | GATE einstellen (20µs) → Zeitfenster für Meßdauer |
> | Spannungsversorgung für Blitzlampe einstellen (800V) → Blitzenergie (ca 1J) |
> | ZÄHLERINHALT löschen |
> | ZÄHLER konfigurieren |
> | SAMPLE/HOLD löschen |
> | ENDE des Unterprogramms |

| WIEDERHOLE bis Messung abgebrochen wird (Eingabe 'q') |
| --- |

> | WIEDERHOLE 4 mal für Filter 1: 760nm, 2: 815nm, 3: 850nm, 4: 870nm |
> | --- |

>> | WIEDERHOLE 50 mal |
>> | --- |

>>> | TRIGGER für Blitz und Messung (Blitz wird gezündet, Messung gestartet) |
>>> | --- |
>>> |     Warten bis Messung beendet ist (Gate beendet) |
>>> | Starte A/D—Wandler, Kanal 0: Referenz |
>>> |     Warten bis Konvertierung beendet ist |
>>> | A/D—Wandler Kanal 0 auslesen: Referenz 'R' |
>>> | Starte A/D—Wandler, Kanal 1: Blitzenergie |
>>> |     Warten bis Konvertierung beendet ist |
>>> | A/D—Wandler Kanal 1 auslesen: Blitzenergie 'B' |
>>> | ZÄHLERINHALT auslesen: M |
>>> | ZÄHLERINHALT löschen |
>>> | SAMPLE/HOLD löschen |

6

## Struktogramm (Fortsetzung)

| Referenz R und Blitzenergie B zulässig? | |
|---|---|
| ja | nein |
| MITTELWERTE für R, B, M | Meßwerte ungültig |
| STANDARTABWEICHUNGEN | |

Nächster Filter

WIEDERHOLE 50 mal (Rausch— und Hintergrundreferenz)

TRIGGER für Messung (Blitz wird _nicht_ gezündet, Messung gestartet)

Warten bis Messung beendet

ZÄHLERINHALT auslesen: M

ZÄHLERINHALT löschen

MITTELWERT, STANDARTABWEICHUNG

| Rauschreferenz zu hoch? | |
|---|---|
| ja | nein |
| Warnung ausgeben, warten bis Taste gedrückt | |

Verarbeitung der Meßwerte einesZyklus:

Rauschreferenz von den Meßwerten abziehen

Meßwerte auf Referenz und Blitzenergie beziehen

Absorptionsänderungen gegenüber Startwerte errechnen

Konzentrationsänderungen für HbO, HbR und C3 berechnen

Konzentrationswerte grafisch darstellen

Meßwerte in gewählter Meßwertedatei abspeichern

ENDE des Unterprogramms

Meßwertedatei schließen

Grafik auf Drucker ausgeben

ENDE des Programms

Die Fig. 6(A) und 6(B) zeigen den optischen Aufbau mit Lichtquelle und umschaltbarem Filterrad. Das Licht der Blitzlampe 1 wird durch eine Konvexlinse 9 auf ein Lichtleitfaserbündel 10 (Durchmesser ca. 5 bis 10 mm) fokussiert. Zur besseren Lichtausbeute ist an der gegenüberliegenden Seite ein Konkavspiegel 11 angebracht. Die optische Achse ist mit Bezugshinweis 12 und die Drehachse des Filterrads 7 mit 13 angegeben. Nach Durchlaufen des Interferenzfilters (z. B. 7a) tritt das Licht in das Faserbündel 10 ein.

Die Prinzipdarstellung der Fig. 7 verdeutlicht den optischen Sender zur Einkopplung des Lichts in das Gewebe. An der Einkoppelstelle 14 des Lichts in das Gewebe wird Licht teilweise reflektiert. Um einen zusätzlichen Korrekturwert für die Auswertung zu erlangen und den Anteil des reflektierten Lichts zu messen, sind direkt neben dem Ort der Einstrahlung vier Photozellen 15a bis 15d angebracht. Über A/D-Wandler, die entweder am Ort der Photozellen direkt oder am Eingang der Sample-Hold-Schaltung 4b vorhanden sein können, werden die Meßwerte in den Rechner 8 eingelesen.

Die Fig. 8(A) und 8(B) zeigen zwei Realisierungen für den optischen Aufnehmer (Empfänger). Um Streulicht aus der Umgebung abzuschirmen, wurde auf der Empfängerseite eine Abdeckmaske 16 mit einem Durchmesser von ca. 50 mm angebracht. Bei dem Aufnehmer nach Fig. 8(A) wird das aus dem Gewebe austretende Licht von einem Lichtleiter 17 aufgenommen und zum Photomultiplier 3a (Fig. 5(A)) weitergeleitet. Als Lichtleiter wurden vier Teilfaserbündel 18 verwendet, die am Photomultiplier 3a zusammengeführt sind.

Bei der Prinzipdarstellung der Fig. 8(B) wird als optischer Sensor eine Mehrzahl von beispielsweise sechs Avalanche-Dioden 19a bis 19f verwendet, deren Signale über ein ge meinsames Kabel 17 auf die Sample-Hold-Schaltung 4b (Fig. 5(B)) geführt sind.

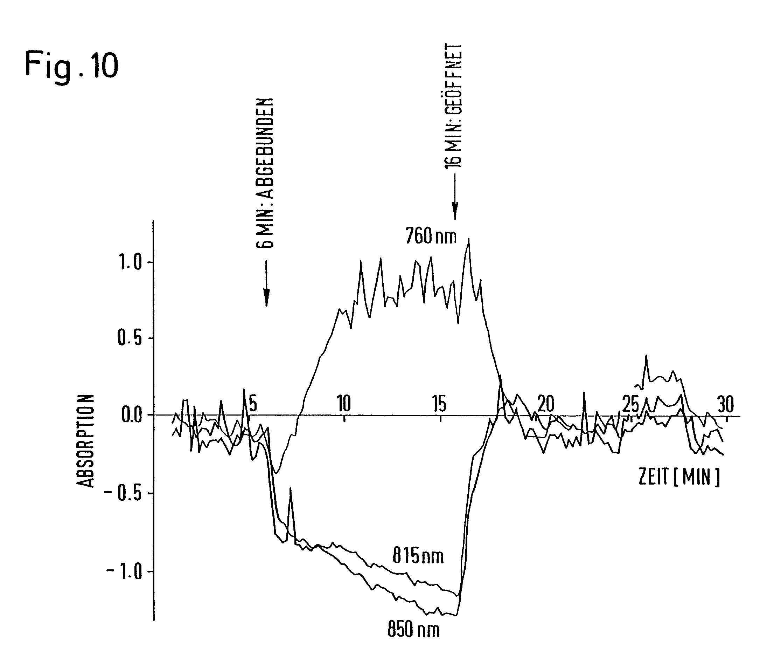

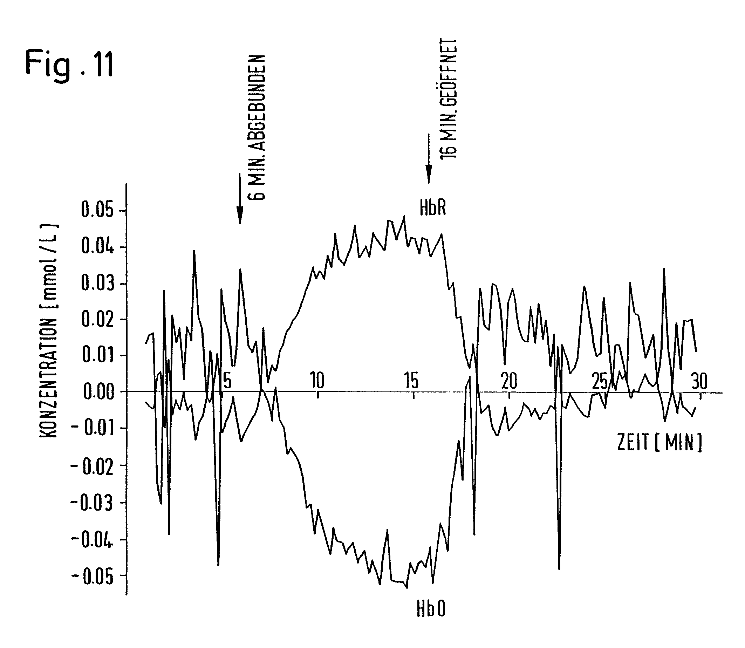

Die Fig. 10 und 11 zeigen praktische, mit dem Systemaufbau nach Fig. 9 gewonnene Meßergebnisse, die die Vorteile der Erfindung augenfällig verdeutlichen.

Die Messungen erfolgten in beiden Fällen am Unterarm eines Probanden. Der Arm wurde, wie durch Pfeil verdeutlicht, einige Minuten lang arteriell abgebunden, d. h. die Blutzufuhr war vollständig unterbrochen. Bei erneuter Blutzufuhr ließen sich die Änderungen der Absorption bei einer Vielzahl von aufgenommenen Meßwerten und den drei oben angegebenen Wellenlängen sehr deutlich ablesen. Ersichtlicherweise ergaben sich Veränderungen der Absorption von mehr als 1.

Die graphische Darstellung der Fig. 11 verdeutlicht die Änderungen der Konzentrationen von oxygeniertem Hämoglobin (HbO) und deoxygeniertem Hämoglobin (HbR) in $\mu$mol/Liter. Auch hier wurden die Werte aus drei gemessenen Absorptionswerten (760nm, 815nm und 850nm) errechnet. Ähnliche Konzentrationsänderungen lassen sich für Cytochrom $aa_3$ in einer graphischen Auswertung darstellen.

**Ansprüche**

1. Spektralphotometer zur Messung am lebenden Organismus, mit
- einer im Pulsbetrieb steuerbaren Lichtquelle (1), die Strahlung im nahen Infrarotbereich abgibt,
- einem ersten Lichtleiter (10), der Licht zu einem zu überwachenden Gewebe leitet,
- einem zweiten Lichtleiter (17), der Licht von dem überwachten Gewebe zu einer Lichtaufnahmeeinrichtung (3a, 3b) leitet und mit
- einer rechnergestützten Steuerungs- und Auswerteeinrichtung (8), welche die pulsweise Erregung der Lichtquelle (1) steuert und mehrere von der Lichtaufnahmeeinrichtung erfaßte gewebesignifikante Wellenlängen der Strahlung auswertet,
**dadurch gekennzeichnet, daß**
- als Lichtquelle (1) eine im wesentlichen mit Krypton gefüllte Gasentladungslampe dient.

2. Spektralphotometer nach Anspruch 1, **dadurch gekenn zeichnet,** daß die Lichtquelle (1) eine mit Krypton gefüllte Blitzlampe ist.

3. Spektralphotometer nach Anspruch 1 oder 2, **dadurch gekennzeichnet,** daß mindestens drei Wellenlängen des Lichts durch die Lichtaufnahmeeinrichtung (16, 18; 19a bis 19f; 3a, 3b) gleichzeitig erfaßt und die entsprechenden Meßsignale gleichzeitig oder im Zeitmultiplex-Abfragebetrieb auf die Auswerteeinrichtung (8) gelangen.

4. Spektralphotometer nach Anspruch 3, **dadurch gekennzeichnet,** daß die Wellenlängen 760 nm, 810 nm und 850 nm als Meßwellenlängen ausgewertet werden.

5. Spektralphotometer nach Anspruch 4, **dadurch gekennzeichnet,** daß zusätzlich bei den Wellenlängen 870 nm und 895 nm gemessen wird.

6. Spektralphotometer nach Anspruch 1 oder 2, **dadurch gekennzeichnet,** daß am gewebeseitigen Ende des ersten Lichtleiters (10) reflektiertes Licht durch wenigstens einen Photodetektor (15a bis 15d) erfaßt und zur Gewinnung eines Referenz- und Korrekturwerts ausgewertet wird.

7. Spektralphotometer nach Anspruch 6, **dadurch gekennzeichnet,** daß mehrere unmittelbar neben dem Ort der Einstrahlung des Lichts in das Gewebe und etwa symmetrisch um das gewebeseitige Ende des ersten Lichtleiters (10) angeordnete Photodetektoren (15a bis 15d), deren jeweilige Signale über A/D-Wandler zur Gewinnung des Referenz- und Korrekturwerts auf die Auswerteeinrichtung (8) gelangen.

8. Spektralphotometer nach Anspruch 1 oder 2, **dadurch gekennzeichnet,** daß als Lichtaufnahmeeinrichtung (3a) ein Photomultiplier verwendet wird und daß das aus dem überwachten Gewebe austretende Licht durch eine Mehrzahl von und vorzugsweise von vier Lichtleitfaserbündeln aufgenommen und zusammengeführt auf den Photomultiplier (3a) geleitet wird.

9. Spektralphotometer nach Anspruch 1 oder 2, **gekennzeichnet durch** eine die Meßstelle am Gewebe abschirmende Abdeckung (16), in die eine Mehrzahl von Avalanche-Dioden (19a bis 19f) eingesetzt ist, die das aus dem Gewebe austretende Licht erfassen und deren Signale über eine Sample-und Hold-Schaltung (4b) auf die Auswerteeinrichtung (8) gelangen.

# Fig.1

# Fig. 2

# Fig. 3

# Fig. 4

WASSERGEKÜHLTE KRYPIONLAMPE KBF 3500 W    100 % ENTSPRECHEN

SPEKTRALE STRAHLSTÄRKEVERTEILUNG

WELLENLÄNGE IN nm

# Fig.5(A)

EP 0 358 809 A1

# Fig.5 (B)

EP 0 358 809 A1

Fig. 6(A)

Fig.6(B)

# Fig . 7

# Fig.8(A)

# Fig.8(B)

Fig. 9

LICHTLEITFASER-BÜNDEL (SENDER)
10

LICHTLEITFASERBÜNDEL (EMPÄNGER)

REFERENZ

| 7;9 | 1 | 20 | 2 |
| OPTIK FILTER | KRYPTON-BLITZLAMPE | KONDENSATOR BATTERIE | SPANNUNGS-VERSORGUNG |

26

PHOTODIODE — PHOTODIODE — MULTICHANNEL-SAMPLE/HOLD — A/D-WANDLER

GATE — DELAY — TRIGGER

PHOTO-MULTIPLIER — VORVER-STÄRKER — KOMPARATOR — ZÄHLER

SPANNUNGS-VERSORGUNG

SYSTEMBUS

RECHNER

MIKROPROZESSOR

SCHREIBER

MONITOR

EP 0 358 809 A1

Fig. 10

Fig . 11

15 151.8
GMBH
EP

# Fig. 12

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | GB-A-2 162 939 (SCLAVO SPA)<br>* ganze Schrift *; & DE - A - 3528369 (Cat. D)<br>--- | 1-9 | G 01 N 21/31<br>A 61 B 5/00 |
| Y | EP-A-0 063 431 (M. KONOMI)<br>* Seiten 1,9, Zeilen 21-23 *<br>--- | 1,2 | |
| Y,D | DE-A-3 019 234 (DUKE UNIVERSITY INC.)<br>* ganze Schrift *<br>--- | 1-9 | |
| A,D | SCIENCE<br>Band 198, 23. Dezember 1977, Seiten 1264-1267; F.F. JOEBSIS: "Noninvasice, Infrared Monitoring of Cerebral and Myocardial Oxygen Sufficiency and Circulatory Parameters".<br>--- | 1,4 | |
| A | MEDICAL & BIOLOGICAL ENGINEERING & COMPUTING<br>Band 26, Nr. 3, Mai 1988, Stevenage, Herts, GB, Seiten 289-294; M. COPE et al.: "System for long-term measurement of cerebral blood and tissue oxygenation on newborn infants by near infra-red transillumination".<br>--- | 1,4-6 | RECHERCHIERTE SACHGEBIETE (Int. Cl.4)<br><br>G 01 N 21/00<br>A 61 B 5/00<br>G 01 J 3/00 |
| A | ABC DER OPTIK, Karl Mütze et al., 1972, Verlag WERNER DAUSIEN, Hanau/Main, DE, Seiten 210,211<br>--- | 2 | |
| A | US-A-4 446 871 (KENJI IMURA)<br>* Spalte 3, Zeilen 19-36; Spalte 4, Zeilen 45-51 *<br>----- | 1,3 | |

Der vorliegende Recherchenbericht wurde für alle Patentansprüche erstellt

| Recherchenort | Abschlußdatum der Recherche | Prüfer |
|---|---|---|
| BERLIN | 08-05-1989 | BRISON O.P. |

EPO FORM 1503 03.82 (P0403)